# NOUVEAU FASCICULE DE BREVET EUROPEEN

(11) **EP 2 735 296 B2**
(45) Date de publication et mention de la décision concernant l'opposition: **06.12.2023**
(45) Mention de la délivrance du brevet: 30.09.2020
(21) Numéro de dépôt: 13193485.3
(22) Date de dépôt: 19.11.2013
(51) Int. Cl.: A61H 7/00, A61H 15/00, A61H 23/02, A61N 5/06, A61N 1/30

(54) **Appareil de massage équipe de têtes de massage interchangeables**
Massagegerät, das mit auswechselbaren Massageköpfen ausgestattet ist
Massage apparatus provided with interchangeable massage heads

(30) Priorité: 22.11.2012 FR 1261107
(43) Date de publication de la demande: 28.05.2014
(73) Titulaire: SEB S.A., 69130 Ecully (FR)
(72) Inventeur: Giraud, Camille, 69001 LYON (FR); Mandica, Franck, 69340 FRANCHEVILLE (FR)
(74) Mandataire: Poindron, Cyrille

(56) Documents cités:
- WO-A1-2010/012857
- CN-U- 201 750 912
- GB-A- 1 021 836
- US-A- 5 103 809
- US-A1- 2008 209 650

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez les ménages composés de personnes désireuses de soigner leur esthétisme en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Parmi l'art antérieur existant, il est notamment connu le brevet EP 1 925 275 B1 qui, outre les caractéristiques précitées, divulgue des moyens d'assemblage et un mécanisme de transmission qui permettent de remplacer facilement la tête de massage pour la changer par une neuve ou différente, la conception du mécanisme de transmission permettant, par ailleurs, d'assembler la tête d'assemblage sur le corps avec un angle d'orientation de l'axe de la tête par rapport à l'axe du corps qui peut être différent. On connait également le document US2008/209650 qui divulgue un appareil comprenant un corps motorisé qui permet d'assembler différents types de tête de brosse à dents, le corps pouvant comprendre des moyens de reconnaissance du type de brosse à dents assemblé sur le corps. Il est également indiqué, en variante, qu'une tête de massage peut être assemblée sur le corps motorisé.

L'objet de la présente invention est de concevoir un appareil de massage de la peau qui permet d'assembler différents types de tête de massage sur le corps de l'appareil de massage, tout en optimisant les performances et les caractéristiques de l'appareil de massage en vue de l'adapter au mieux en fonction du type de tête de massage utilisé. A cet effet, l'invention est définie dans les revendications indépendantes 1, 2, 3, 4 et 7.

L'invention concerne un appareil de massage comprenant un corps qui comporte des moyens de motorisation formés par un moteur alimenté électriquement, au moins un type de tête de massage qui comprend des éléments de massage, un mécanisme de transmission qui permet l'activation des éléments de massage sous l'action des moyens de motorisation et, des moyens d'assemblage qui sont configurés pour assembler de manière amovible l'au moins un type de tête de massage avec le corps. Ainsi l'appareil de massage selon l'invention permet de remplacer facilement et rapidement la tête de massage par une autre identique ou différente. Selon l'invention, l'appareil comprend des moyens de reconnaissance du type de tête de massage assemblé sur le corps et des moyens de commande dudit appareil de massage en fonction de la reconnaissance du type de tête de massage. Ainsi, l'appareil permet de détecter le type de tête assemblé sur le corps et s'adapte en fonction du type de tête de massage, par exemple en adaptant la vitesse des moyens de motorisation, en commandant l'un ou l'autre des éléments des moyens de motorisation, voire autres. Cela présente pour avantage de pouvoir adapter des têtes de massage présentant des caractéristiques de fonctionnement bien différentes entre elles.

Selon un premier mode de réalisation de l'appareil de massage objet de l'invention, les moyens de reconnaissance sont des capteurs mécaniques agencés sur l'au moins un type de tête de massage et le corps et, configurés pour transmettre des informations aux moyens de commande en fonction du type de tête de massage assemblé sur le corps. Les capteurs mécaniques couvrent tout capteur mécanique y compris des contacteurs électriques qui viendraient en contact l'un avec l'autre lorsque la tête de massage est assemblée sur l'appareil.

Selon un second mode réalisation de l'appareil de massage objet de l'invention, les moyens de reconnaissance sont des capteurs magnétiques agencés sur l'au moins un type de tête de massage et le corps et, configurés pour transmettre des informations aux moyens de commande en fonction du type de tête de massage assemblé sur le corps.

Selon un troisième mode de réalisation de l'appareil de massage objet de l'invention, les moyens de reconnaissance sont des capteurs optiques agencés sur l'au moins un type de tête de massage et le corps et, configurés pour transmettre des informations aux moyens de commande en fonction du type de tête de massage assemblé sur le corps.

Bien entendu, d'autres modes de réalisation des moyens de reconnaissance sont envisageables sur l'appareil de massage, sans sortir du cadre de l'invention.

Selon l'appareil de massage objet de l'invention, les moyens de commande sont configurés pour agir sur les moyens de motorisation et modifier leur actionnement en fonction du type de tête de massage assemblé sur le corps. Ainsi, par exemple, la vitesse d'activation des éléments de massage de la tête de massage peut être adaptée, ce qui permet d'optimiser les conditions d'utilisation de la tête de massage. Cela permet également d'agencer sur le corps des moyens de motorisation plus ou moins complexes, configurés pour actionner différents types de tête de massage présentant des caractéristiques de conception très diverses et de commander ces moyens de motorisation en fonction de la reconnaissance de la tête de massage.

Selon l'appareil de massage objet de l'invention, celui-ci comprend un système d'émission d'ondes agencé sur le corps et des moyens de transfert des ondes, du système d'émission vers la tête de massage assemblée sur le corps. Cela permet de disposer de fonctions de traitement de la peau complémentaires à celles des têtes de massage. Ces ondes peuvent être des ondes électromagnétiques, particulièrement lumineuses, visibles ou dans le domaine de l'infrarouge, ou peuvent être sonores par exemple des ultrasons. Cela permet également de travailler de manière complémentaire, la peau de l'utilisateur. Dans un exemple envisagé de réalisation, ces ondes sont lumineuses de couleur rouge ou orange. Dans un mode de réalisation préférentiel mais non limitatif, l'appareil de massage comprend un système luminescent agencé sur le corps et des moyens de transfert de la lumière, du système luminescent vers la tête de massage.

Selon l'appareil de massage objet de l'invention, les moyens de commande sont configurés pour agir sur le système d'émission d'ondes et modifier les ondes en fonction du type de tête de massage assemblé sur le corps.

Selon l'appareil de massage objet de l'invention, celui-ci comprend un système de distribution de produit cosmétique agencé sur le corps et des moyens de transfert du produit, du corps vers la tête de massage. Cela permet également de disposer de fonctions de traitement de la peau complémentaires à celles des têtes de massage. Selon la configuration du système de distribution, la distribution du produit cosmétique pourra être réalisée de manière naturelle, manuelle et/ou automatique.

Dans un mode de réalisation, l'appareil de massage selon l'invention comprend des moyens d'actionnement du système de distribution de produit cosmétique, configurés pour permettre le fonctionnement dudit système de distribution en mode manuel ou en mode automatique. Cela permet de laisser toute liberté d'utilisation de l'appareil de massage ; l'utilisateur peut ainsi choisir de réaliser un traitement cosmétique en complément de celui obtenu avec la tête de massage.

Selon l'appareil de massage objet de l'invention, les moyens de commande sont configurés pour agir sur le système de distribution de produit cosmétique et modifier la distribution du produit cosmétique en fonction du type de tête de massage assemblé sur le corps. Cela permet de réaliser un traitement cosmétique adapté aux différentes têtes de massage.

Selon l'appareil de massage objet de l'invention, les moyens d'assemblage comprennent un système de détection de l'assemblage convenable de la tête de massage sur le corps. Cela permet de garantir une utilisation de l'appareil de massage dans de bonnes conditions.

Selon l'appareil de massage objet de l'invention, celui-ci comprend des moyens de connexion entre les moyens de motorisation et le mécanisme de transmission configurés pour s'affranchir de l'angle d'orientation du mécanisme de transmission par rapport au moyen de motorisation, lors de l'assemblage de la tête de massage sur le corps. Ainsi, il est possible d'assembler des têtes de massage sur le corps avec des orientations différentes, certaines têtes de massage pouvant par exemple être positionnées dans le prolongement du corps lors de l'assemblage, tandis que d'autres têtes sont positionnées avec une inclinaison à 45° par rapport au corps.

Selon un mode de réalisation de l'appareil de massage objet de l'invention, un des types de tête de massage comporte une surface d'application et les éléments de massage sont constitués d'au moins deux embouts de massage qui s'étendent perpendiculairement vers l'extérieur de la surface d'application, les embouts de massage étant définis sur la surface d'application selon des cercles virtuels concentriques de centre C et, dans lequel mécanisme de transmission est configuré pour rapprocher les embouts de massage et/ou, inversement, pour écarter les embouts de massage, en translatant lesdits embouts de massage dans un sens et/ou dans l'autre selon des trajectoires qui se rejoignent au centre C. Ce type de tête de massage permet de réaliser un pincé de la peau.

Selon un mode de réalisation de l'appareil de massage objet de l'invention, un des types de tête de massage comporte une surface d'application et les éléments de massage sont constitués de deux embouts de massage qui s'étendent perpendiculairement vers l'extérieur de la surface d'application, le mécanisme de transmission étant configuré pour actionner en rotation selon un axe perpendiculaire à la surface d'application, avec un mouvement oscillatoire, les deux embouts. Ce type de tête de massage permet de réaliser un travail avec précision autour des yeux et de la bouche, notamment, en appliquant les embouts de massage perpendiculairement à la peau pour réaliser un effet pincé tournant ou, parallèlement à la peau pour réaliser un effet tenseur remodelant.

Selon un mode de réalisation de l'appareil de massage objet de l'invention, la tête de massage comporte une surface d'application et les éléments de massage sont constitués de trois embouts de massage inclinés vers l'extérieur par rapport à la surface d'application, le mécanisme de transmission étant configuré pour actionner en rotation ou en oscillation, les trois embouts de massage selon trois axes fixes respectifs, perpendiculaires à la surface d'application. Ce type de tête de massage permet de réaliser un léger pincé de la peau.

Selon un mode de réalisation de l'appareil de massage objet de l'invention, la tête de massage comprend une surface d'application et les éléments de massage sont constitués d'au moins deux doigts de massage qui s'étendent vers l'extérieur de la surface d'application, les doigts de massage et le mécanisme de transmission étant configurés pour former une pince. Ce type de tête de massage permet de réaliser un pincé de la peau.

Selon ces divers modes de réalisation de l'appareil de massage objet de l'invention, avec des embouts de massage ou des doigts de massage, la tête de massage comprend une peau souple qui recouvre les éléments de massage. Cela évite qui les embouts de massage ou les doigts de massage ne paraissent trop agressif et permet de mieux agripper la peau grâce à un contact surfacique sur la peau.

Selon un mode de réalisation de l'appareil de massage objet de l'invention, la tête de massage comprend une couronne s'étendant perpendiculairement à la surface d'application et, le mécanisme de transmission est configuré pour entraîner la couronne avec un mouvement oscillatoire autour d'un axe perpendiculaire à la surface d'application. Cela permet de bien agripper la peau et de réaliser des mouvements complexes sur la peau, en combinaison avec des embouts de massage ou des doigts de massage.

Selon un mode de réalisation de l'appareil de massage objet de l'invention, la tête de massage comprend une surface d'application et les éléments de massage sont constitués de deux rouleaux de massage agencés selon deux axes longitudinaux parallèles, avec un écartement entre eux, et dépassant partiellement à l'extérieur de la surface d'application et, dans lequel mécanisme de transmission est configuré pour entraîner en rotation de manière synchronisée et inversée, les deux rouleaux de sorte que, dans une vue en plan perpendiculaire aux deux axes, la partie du rouleau de massage située à gauche qui dépasse de la surface d'application, tourne dans le sens trigonométrique et, la partie du rouleau de massage située à droite qui dépasse de la surface d'application, tourne dans le sens horaire. Ce type de tête de massage permet de réaliser un plissage de la peau.

Selon l'appareil de massage objet de l'invention, celui-ci comprend un dispositif de traitement ionophorèse transcutanée qui est configuré pour transmettre sur la peau lors de l'application dudit appareil de massage, un courant permettant d'augmenter et/ou d'accélérer la pénétration d'un produit cosmétique qui peut être soit distribué par le système de distribution de produit cosmétique décrit ci-dessus, soit appliqué directement sur la peau par l'utilisateur. L'ionophorèse est un traitement qui a été développée initialement pour l'application dans la peau de médicaments, en particulier pour de la médecine sportive, mais est également envisagée maintenant pour une meilleure pénétration d'un cosmétique. Dans un mode de réalisation, ce dispositif de traitement ionophorèse transcutanée comprend deux électrodes à potentiel électrique différent qui peuvent être agencées sur des embouts ou rouleaux de massage et/ou sur la surface d'application de la tête de massage. Les réglages de l'appareil de massage selon l'invention, en particulier lorsque celui-ci comprend également un système de distribution de produits cosmétique, permettront de réaliser le traitement par ionophorèse transcutanée avant et /ou pendant l'application du produit cosmétique. Ces réglages dépendront de la reconnaissance de la tête de massage assemblée sur le corps.

La description suivante met en évidence les caractéristiques et avantages de la présente invention, laquelle s'appuie sur des figures parmi lesquelles :
- La figure 1 schématise un appareil de massage selon l'invention pouvant recevoir différentes conceptions de têtes de massage ;
- La figure 2 schématise une tête de massage comprenant des rouleaux de massage ;
- La figure 3 schématise une tête de massage comprenant des embouts de massage ;
- La figure 4 schématise un système d'assemblage amovible entre la tête de massage et le corps ;
- Les figures 5 et 6 schématisent une tête de massage comprenant trois embouts inclinés ;
- La figure 7 schématise une couronne sur la tête de massage ;
- La figure 8 schématise une peau souple sur la tête de massage ;
- Les figures 9 et 10 schématisent un mécanisme de transmission entre la tête de massage et le corps ;
- La figure 11 schématise un système de connexion par fibre optique entre la tête de massage et le corps ;
- Les figures 12 et 13 schématisent une variante de tête de massage munie de pinces ;
- Les figures 14 à 16 schématisent des variantes de moyens de reconnaissance de la tête de massage sur le corps ;
- Les figures 17 à 20 schématisent des variantes de moyens de reconnaissance de la tête de massage sur le corps par capteurs optiques ;
- Les figures 21 et 22 schématisent un système de distribution de produit cosmétique ;
- Les figures 23 et 24 schématisent une variante de système de distribution de produit cosmétique ;
- La figure 25 schématise un système vibratoire sur l'appareil de massage ;
- La figure 26 schématise des diodes luminescentes sur la tête de massage ;
- La figure 27 schématise un dispositif de traitement ionophorèse transcutanée sur l'appareil de massage ;
- La figure 28 illustre un système de distribution agencé entre le corps et la tête de massa ;
- Les figures 29 à 36 illustrent des variantes de réalisation de système d'encliquetage entre la tête de massage et le corps ;
- La figure 37 schématise la reconnaissance de différentes têtes de massage lors de leur assemblage sur le corps.

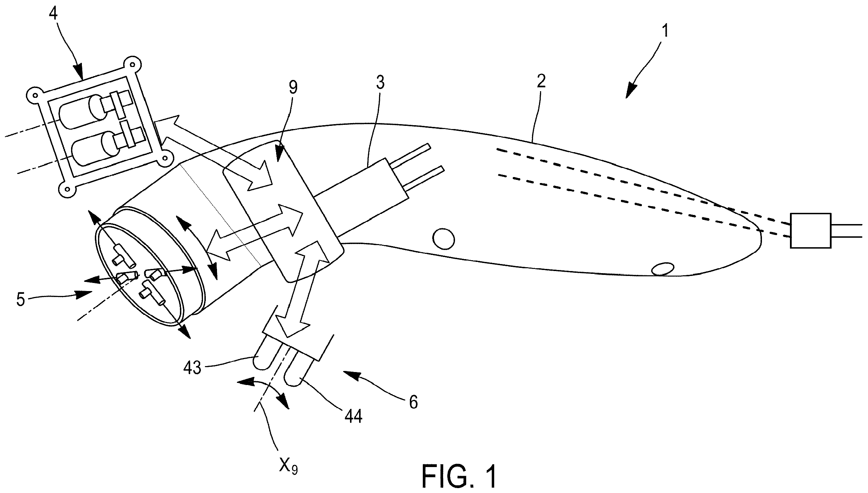

Tel qu'illustré sur la figure 1, l'appareil de massage 1 comprend un corps 2 à l'intérieur duquel est agencé un moteur 3 alimenté électriquement par raccordement soit à une source électrique externe, au moyen d'une prise électrique et d'un transformateur basse tension, soit à source électrique interne du type batterie rechargeable ou piles jetables. L'appareil de massage 1 comprend également un moto réducteur agencé en sortie du moteur 3 pour réduire sa vitesse de rotation et l'adapter au besoin d'utilisation. L'appareil de massage 1 est configuré pour permettre la connexion de différents types de tête de massage 4, 5, 6, 7, 8 illustrés sur les figures 1, 2, 3, 5, 6, 12 et 13. Pour cela, l'appareil de massage comprend des moyens de connexion amovible 9 entre le corps 2 et la tête de massage 4, 5, 6, 7, 8.

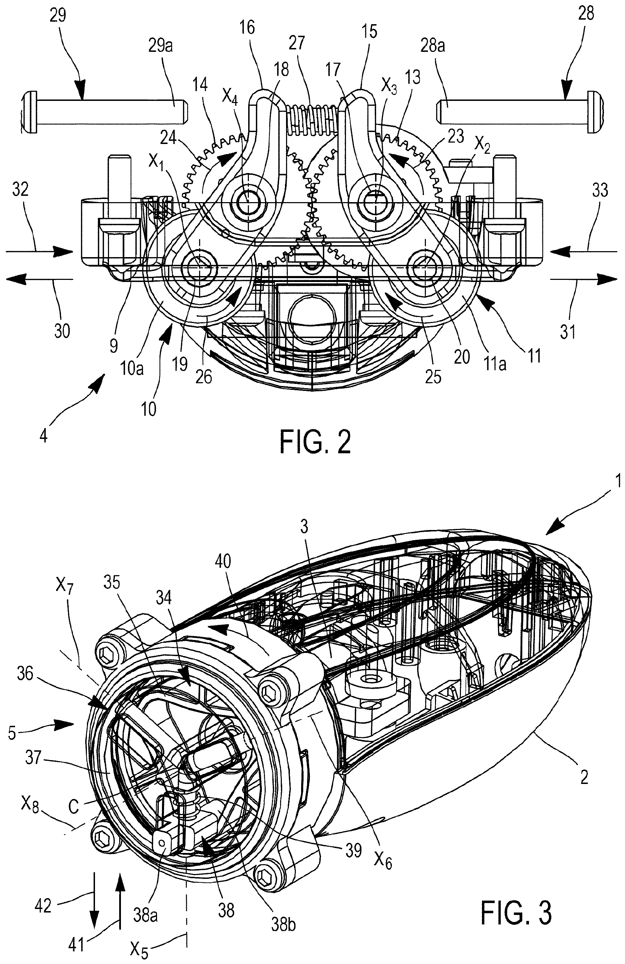

Sur la figure 2, la tête de massage 4 comprend une surface d'application 9 et deux rouleaux de massage 10, 11 dont une partie 10a, 11a dépasse de la surface d'application 9. Ces rouleaux 7, 8 sont disposés selon deux axes X₁, X₂ parallèle entre eux. Selon ce mode de réalisation, le moteur 3 comprend un arbre d'entraînement assujetti à une roue dentée 13. Ainsi le moteur 3 entraîne en rotation la roue dentée 13 selon un axe X₃. Cette roue dentée 13 engrène avec une autre roue dentée 14 de conception identique et disposée selon un axe X₄.

Tel qu'illustré sur la figure 2, la tête de massage 4 comprend deux potences 15, 16. Ces potences 15, 16 sont montées en liaison pivot d'axes respectifs X₃, X₄ au moyen de deux arbres 17, 18. Ces potences reçoivent en liaison pivot d'axe X₁, X₂ les rouleaux 10, 11 au moyen de deux arbres 19, 20 montés en liaison pivot sur lesdites potences 15, 16.

Cette tête de massage 4 comprend deux autres roues dentées de conception identique (non illustrées). L'une des roues dentées est assujettie au rouleau 11 par le biais de l'arbre 20, par exemple au moyen de cannelures (non illustrées) agencées entre ces éléments, et engrène avec la roue dentée 13. De même, l'autre roue dentée est assujettie au rouleau 10 par le biais de l'arbre 19 et, engrène avec la roue dentée 14. Ainsi, lors de l'entraînement de la roue dentée 13 par le biais du moteur 3, qui tourne dans le sens de la flèche 23, la roue dentée 14 tourne dans le sens de la flèche 24, ce qui permet d'entraîner le rouleau 10 est entraîné dans le sens de la flèche 26 et le rouleau 11 dans le sens de la flèche 25.

Tel qu'illustré sur la figure 2, des ressorts 27 sont agencés entre les deux potences 15, 16. En position initiale, ces ressorts 27 sont actifs contre les potences 15, 16 et les maintiennent en appui contre les extrémités 28a, 29a de deux vis de réglage 28, 29 agencées sur la tête de massage 4. Ainsi les rouleaux 10 ou 11 sont écartés l'un de l'autre avec un écartement minimal en position initiale. En outre, le réglage des vis 28, 29 permet de modifier l'écartement minimal entre ces deux rouleaux 7, 8. Lorsque les parties 10a, 11a des rouleaux 10, 11 sont appliquées sur la peau, les sens des rotations inversés des rouleaux 10, 11 dans le sens des flèches 25, 26 permet de former un pli sur la peau. Les efforts exercés par la peau sur ces rouleaux, lorsque ces efforts sont supérieurs aux efforts exercés par les ressorts 27, permettent alors de comprimer ces ressorts 27 et d'écarter les rouleaux dans le sens des flèches 30, 31 illustrées en figure 2. Au contraire, si ces efforts exercés par la peau sur les rouleaux 10, 11 sont inférieurs aux efforts exercés par les ressorts 27, ces ressorts 27 se détendent et ramènent les rouleaux 10, 11 dans le sens des flèches 32, 33 illustrées en figure 2, jusqu'à atteindre une position d'équilibre.

Des variantes de réalisation de mécanismes de transmission sur la tête d'assemblage 4, fonctionnant selon le même principe, sont envisageables sans sortir du cadre de l'invention.

Tel qu'illustré en figure 3, la variante de tête de massage 5 comprend une pièce de transmission 34 qui comporte une came 35 de forme triangulaire. Une pièce de guidage 36, de forme cylindrique, présente une surface d'application 37 qui est positionnée parallèlement à la surface de la peau lors de l'utilisation de l'appareil de massage 1.

Sur cette figure 3, est illustré un seul embout de massage 38. On comprend toutefois en regard de cette figure 3, que la tête de massage 5 selon ce mode de réalisation comprend trois embouts de massage uniformément répartis et identiques. L'embout de massage 38 comprend une portion externe 38a qui s'étend perpendiculairement à la surface d'application 37, vers l'extérieur de celle-ci. Cette portion externe 38a vient en contact avec la peau de l'utilisateur lors de l'application de l'appareil de massage 1.

Tel qu'illustré sur la figure 3, la pièce de guidage 36 permet de monter en translation selon l'axe X₅ sur la pièce de guidage 36. Il en est de même pour les embouts selon les axes X₆ et X₇. Le moteur 3 permet l'entraînement en rotation selon l'axe X₈ de la pièce de guidage 36.

Tel qu'illustré en figure 3, l'embout de massage 38 comprend une portion interne 38b. Lorsque la tête de massage 5 est assemblée sur le corps 2, cette portion interne 38b est positionnée à l'intérieur de la came 35 de la pièce de transmission 9. Cette portion interne 38b est maintenue en appui contre la came 35 au moyen d'un ressort 39. Lorsque la pièce de guidage 36 est entraînée en rotation selon l'axe X₈ dans le sens de la flèche 40, l'embout de massage 38 tourne avec celle-ci. La came 35 exerce un effort contre la portion interne 69 et pousse l'embout de massage 38 dans le sens de la flèche 41 sur une première portion angulaire, ce qui permet de translater l'embout de massage 21 selon l'axe X₅ dans le sens de la flèche 41, vers le centre C défini par l'intersection de l'axe X₈ avec les axes X₅, X₆, X₇. Puis, sur une seconde portion angulaire, la came 35 cesse d'exercer cet effort sur la portion interne 38b. Le ressort 39 qui exerce une fonction de retour, pousse contre de l'embout de massage 38 dans le sens de la flèche 42 et le translate selon l'axe X₅ dans le sens de cette flèche 42, pour l'éloigner du centre C. La rotation de la pièce de guidage 36 autour de l'axe X₈ combiné à la translation de l'embout de massage 38 selon l'axe X₅ permet donc à cet embout de massage 38 de se rapprocher puis de s'éloigner du centre C en effectuant un mouvement de translation circulaire ou curviligne. On comprend que les deux autres embouts de massage subissent les mêmes mouvements en synchronisation avec l'embout de massage 38. Ainsi les trois embouts de massage 38 sont disposés sur un même cercle virtuel de centre C et se déplacent en effectuant une translation curviligne passant par le centre C. Cela permet de réaliser un pincé tournant de la peau au moyen de la tête de massage 5.

Des variantes de réalisation à quatre embouts sont envisageables pour la tête de massage 5. On peut également prévoir une pièce guidage fixe et une pièce de transmission tournant autour de l'axe X₈, dans quel cas les embouts de massage translateront radialement vers le centre C pour s'éloigner ou se rapprocher de celui-ci. On peut également envisager de multiples variantes de cames en fonction du nombre d'embouts présent.

D'autres variantes de têtes de massage sont envisageables. Sur la figure 1 on constate que la tête de massage 6 comprend deux embouts 43, 44 qui sont entraînés en rotation ou en oscillation autour d'un axe Xg par le biais du moteur 3 et d'un mécanisme de transmission.

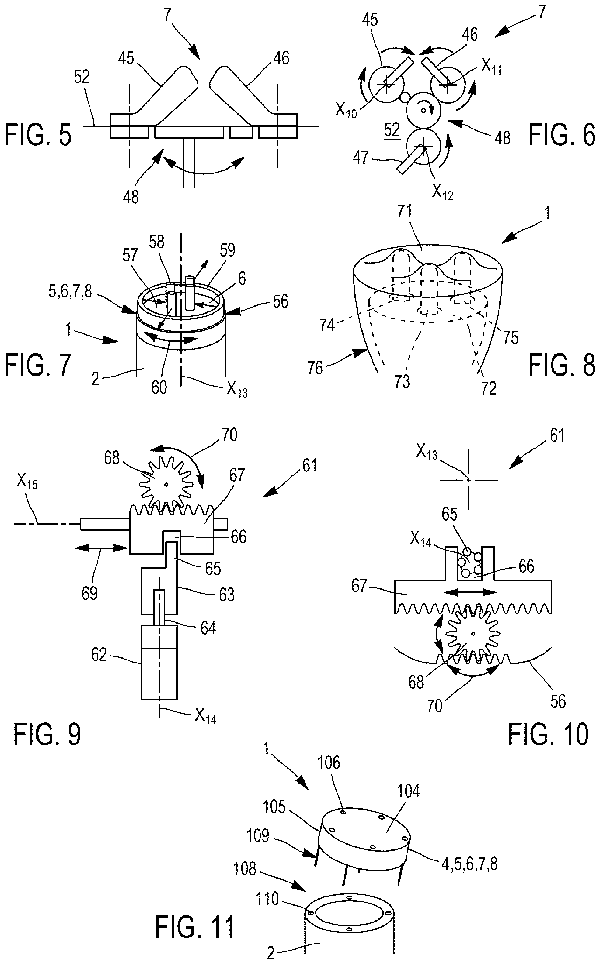

Sur les figures 5 et 6, la tête de massage 7 comprend trois embouts de massage 45, 46, 47 entraînés en rotation autour des axes X₁₀, X₁₁, X₁₂ par un mécanisme d'engrenage 48. Ces embouts de massage 45, 46, 47 sont inclinés par rapport à la surface d'application 52.

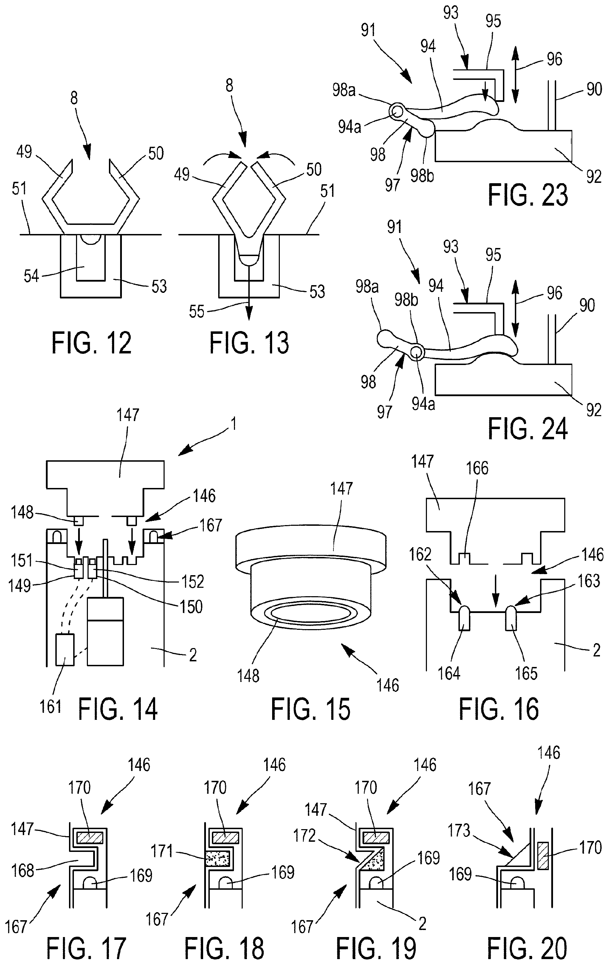

Sur les figures 12 et 13, la tête de massage 8 comprend deux pinces 49, 50 qui s'étendent vers l'extérieur de la surface d'application 51. Ces pinces 49, 50 sont souples. La tête de massage 8 comprend une pièce rigide 53 avec une chambre 54 dans laquelle peuvent pénétrer les pinces 49, 50 qui se déforment du fait de leur souplesse et se ferment. La translation des pinces 49, 50 à l'intérieur de la chambre 54 est assurée par un mécanisme de traction dans le sens de la flèche 55. La souplesse des pinces 49, 50 permet leur retour en position écartée lorsque le mécanisme de traction autorise le déplacement de ces pinces 49,50 dans le sens inversé à la flèche 55.

Dans une variante illustrée sur la figure 7, l'appareil de massage 1 comprend une couronne 56 qui peut être agencée avec les têtes de massage 5, 6, 7, 8 à embouts ou à pinces. Sur la figure 7, des embouts 58 sont représentés. Cette couronne 56 est entraînée en rotation selon l'axe X₁₃ et s'étend par rapport à la surface d'application 57 de sorte que les extrémités des embouts de massage 58 soient positionnées dans le même plan que le bord périphérique 59 de la couronne 56. Cette couronne 56 est de préférence actionnée en rotation selon un mouvement alternatif d'axe X₁₃ dans les sens de la flèche 60. Pour cela, l'appareil de massage 1 comprend un mécanisme de transmission 61 illustré en figure 9 et 10. Ce mécanisme de transmission 61 comprend un moto réducteur 62 qui est disposé dans le corps 2 de l'appareil de massage 1. Ce moto réducteur 62 peut être différent ou le même que le moto réducteur du moteur 3. Il peut en outre être entraîné par le même moteur 3 par le biais d'un système d'engrenage (non illustré) voire par un moteur indépendant disposé dans le corps 2. Un excentrique 63 est agencé sur l'arbre 64 du moto réducteur 62. Cet excentrique 63 comprend un doigt 65 décalé par rapport à l'axe X₁₄ de rotation de l'arbre 64. Ce doigt 65 est positionné dans une rainure 66 agencée sur une crémaillère 67 montée en translation selon un axe X₁₅ et engrenant avec un pignon 68 qui engrène lui-même avec la couronne 56, comme illustré en figures 9 et 10. Ainsi le doigt 65 dispose d'un certain degré de liberté dans la rainure 66 lors de sa rotation autour de l'axe X₁₄, ce qui permet de transmettre un mouvement alternatif selon la flèche 69 à la crémaillère et donc, un mouvement de rotation alternatif selon la flèche 70 au pignon 68 et à la couronne 56. L'avantage de ce mécanisme transmission 61 est de pouvoir modifier facilement l'angle d'inclinaison entre le doigt 65 et la rainure 66, ce qui permet d'assembler la tête de massage sur le corps 2 avec différentes orientations angulaires, par exemple dans l'axe du corps 2 ou avec une inclinaison à 45 degrés par rapport au corps 2. On obtient ainsi des moyens de connexion qui permettent de s'affranchir des angles entre la tête de massage et le corps. Une telle connexion peut être utilisée avec des variantes de mécanisme de transmission entre la tête de massage et le corps, selon le besoin. On peut également envisager des variantes de connexion qui permettent de s'affranchir de l'angle d'inclinaison entre la tête de massage et le corps, comme par exemple, un système d'engrenage à roue conique ou équivalent.

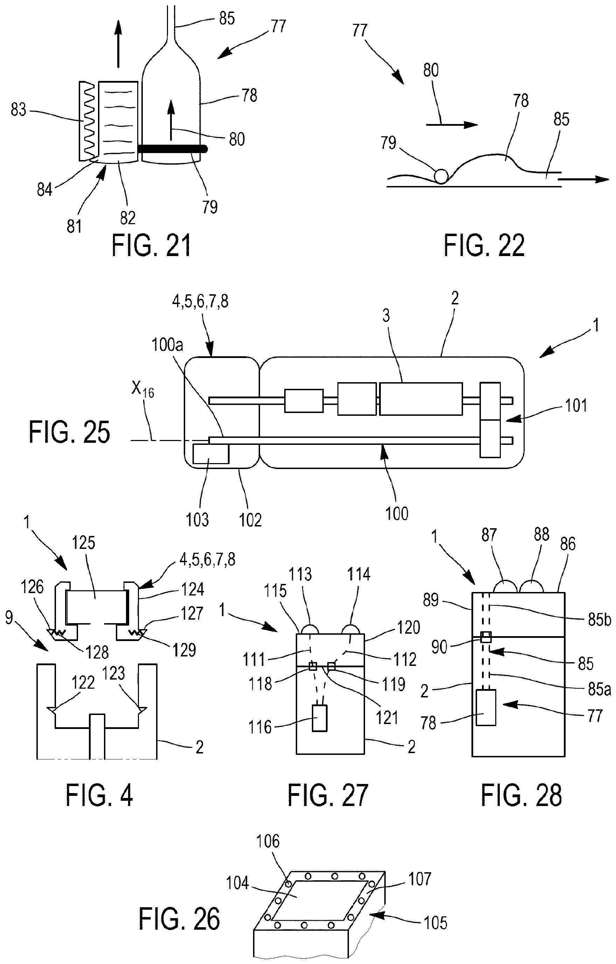

Dans une variante illustrée sur la figure 28, l'appareil de massage 1 comprend une peau souple 71 qui est agencée au-dessus de la surface d'application 72 de la tête de massage 76 et recouvre des embouts de massage 73, 74, 75. Cette peau souple 71 évite notamment que les embouts ne paraissent trop agressifs et améliore l'adhérence sur la peau grâce à un contact surfacique. On peut envisager cette peau souple avec les pinces 49, 50 des figures 12 et 13 ou les diverses variantes à embouts, voire d'autres variantes.

Dans une variante illustrée en figures 21 et 22, on prévoit sur l'appareil de massage 1 un système de distribution de produit cosmétique 77 qui comprend un réservoir de produit 78 souple sur lequel appuie une barrette 79 qui se déplace en translation selon la flèche 80 le long du réservoir de produit 78. Un système d'actionnement 81 permet de déplacer la barrette 79 selon la flèche 80. Ce système d'actionnement 81 est par exemple constitué d'un bouton 82 solidaire de la barrette 79 et translatant dans le sens de cette flèche 80 lors d'une action manuelle. Une pièce crantée 83 permet de maintenir un cran 84 sur le bouton 82 lors de chaque impulsion sur celui-ci pour maintenir en position la barrette 79 au fur et à mesure de son avancement sur le réservoir de produit 78. Le système de distribution 77 comprend un tuyau 85 dont l'agencement permet de distribuer le produit cosmétique au niveau de la surface d'application 86, soit entre les éléments de massage 87, 88, soit au travers des éléments de massage 87, 88, comme illustré en figures 28. Ces éléments de massage 87, 88 sont des rouleaux de massage sur la figure 28 ; ceux-ci peuvent bien entendu être des embouts ou des pinces. On constate sur la figure 28 que le tuyau 85 comprend une première portion 85a disposée dans le corps 2 de l'appareil de massage et, une seconde portion 85b disposée dans la tête de massage 89. Il est prévu des moyens de raccordement étanche 90 entre la première portion 85a et la seconde portion 85b du tuyau. Divers moyens de raccordement étanche 90 pourront être envisagés, par exemple une connexion mâle et femelle munie de joints d'étanchéité.

D'autres variantes de système de distribution de produit cosmétique sont envisageables. On peut notamment prévoir un système d'actionnement automatique permettant de distribuer de manière continue et régulière, par exemple au moyen d'une pompe, par exemple pompe électrique, le produit cosmétique. On peut également prévoir un système de distribution de produit cosmétique de manière naturelle, le produit cosmétique étant diffusé régulièrement grâce à des phénomènes physiques ne nécessitant pas un actionnement extérieur.

Dans une variante illustrée en figures 23 et 24, le système de distribution 91 comprend un réservoir 92 qui est souple et un actionneur 93 qui comprend une manette 94 et un doigt motorisé 95 configuré pour se déplacer selon un mouvement alternatif selon les flèches 96. Le système de distribution 91 comprend un système de glissière 97 qui présente une lumière 98 dans laquelle peut se déplacer l'extrémité arrière 94a de la manette 94. La manette 94 est configurée pour être placée dans une première position illustrée en figure 23, selon laquelle l'extrémité arrière 94a de la manette 94 est positionnée à l'arrière 98a de la lumière et ladite manette 94 est dégagée du doigt motorisé 95. Ainsi, il est nécessaire d'exercer une impulsion manuelle sur la manette 94 pour appuyer sur le réservoir 92 et distribuer du produit cosmétique au travers du tuyau 99 qui pourra présenter une configuration similaire au tuyau 85 sur la figure 28. Au contraire, lorsque l'extrémité arrière 94a de la manette 94 est positionnée à l'avant 98b de la lumière, tel qu'illustré en figure 24, le doigt motorisé 95 actionne automatiquement la manette 94 qui presse le réservoir 92 et distribue le produit cosmétique au travers du tuyau 99. Des variantes de réalisation d'un système de distribution de produit cosmétique avec un système de basculement en mode manuel ou en mode automatique, peuvent être envisagées sans sortir du cadre de l'invention.

Tel qu'illustré en figure 25, l'appareil de massage 1 comprend un arbre 100 qui est entraîné en rotation selon un axe X₁₆ par le moteur 3, au moyen d'un système d'engrenage 101 connu de l'homme du métier. L'extrémité 100a de cet arbre 100 est agencée dans la tête de massage 4, 5, 6, 7, 8, par exemple sur le carter 102 sur les éléments de massage, et reçoit une masselotte 103 qui tourne en balourd autour de l'axe X₁₆ et permet de faire vibrer la tête de massage durant l'application de l'appareil de massage 1. D'autres variantes de système vibratoire restent envisageables. On peut notamment envisager de disposer la masselotte 103 directement dans le corps 2 à proximité de la tête de massage, pour simplifier la conception et éviter la présence d'un système de connexion supplémentaire sur l'arbre 100 au niveau de l'assemblage entre le corps et la tête de massage. On peut également prévoir d'intégrer complètement le système vibratoire dans la tête de massage, un arbre d'entraînement activé par le moteur 3 ou un autre moteur, pénétrant dans la tête, un système de connexion comparable à celui des figures 9 et 10 pouvant alors être envisagé, par exemple.

Dans une variante illustrée en figures 11 et 26, l'appareil de massage 1 comprend au niveau de la surface d'application 104 sur la tête de massage 105, des diodes luminescentes 106 qui sont commandées par un boîtier électronique (non illustré) agencé à l'intérieur du corps 2. Ces diodes luminescentes 106 pourront être allumées soit automatiquement lors de l'actionnement de la tête de massage 4, 5, 6, 7, 8, soit séparément au moyen du bouton de commande distinct (non illustré). Les diodes luminescentes 106 seront disposées par exemple sur le bord périphérique 107 de la surface d'application 104, voire réparties sur ladite surface d'application 104 en dehors des trajectoires des éléments de massage. On pourra utiliser des diodes luminescentes de différentes couleur ou multi-couleurs, selon la longueur d'onde souhaitée et/ou le traitement recherché, voire prévoir un boîtier de commande permettant de modifier la longueur d'onde de ces diodes luminescentes 106. Un système de transmission de la lumière 108 est agencé entre la tête de massage 4, 5, 6, 7, 8 et le corps 2. Ce système de transmission de la lumière comprend une connexion par fibres optiques 109, 110 qui permet de diriger la lumière émise vers la surface d'application 104 ou sur les éléments de massage tels que des embouts de massage.

Dans une variante de réalisation illustrée en figure 27, l'appareil de massage 1 comprend deux électrodes 111, 112 qui présentent un potentiel électrique différent. Ces électrodes 111, 112 peuvent consister en deux éléments de massage 113, 114 ou être agencées sur la surface d'application 115. Ces électrodes 111, 112 sont alimentées par une source électrique 116 agencé dans le corps 2. Cette conception permet de réaliser un traitement par ionophorèse transcutanée avant ou pendant l'application d'un produit cosmétique sur la peau, ce qui permet d'accélérer la pénétration du produit cosmétique. Ce produit cosmétique peut être distribué de manière naturelle, manuelle ou automatique par l'appareil de massage 1, voire appliqué directement sur la peau par l'utilisateur. L'appareil de massage 1 comprend des moyens de connexion amovibles 117, 118 qui permettent de déconnecter ou de connecter rapidement les électrodes 111, 112 lors du démontage ou du montage de la tête de massage 120 sur le corps 2. On peut par exemple prévoir des contacteurs électriques au niveau de la zone d'assemblage 121 entre la tête de massage 120 et le corps 2.

Tel qu'illustré au regard des figures 1 et 4, l'appareil de massage 1 comprend des moyens d'assemblage 9 qui sont configurés pour que la tête de massage 4, 5, 6, 7, 8 puisse être montée et démontée rapidement du corps 2. Ces moyens d'assemblage 9 sont mis en œuvre au moyen d'un système de fixation amovible entre la tête de massage et le corps. Selon le mode de réalisation sur la figure 4, le corps 2 comprend des encoches 122, 123 et la tête de massage 4, 5, 6, 7, 8 comprend une pièce 124 qui reçoit le mécanisme de transmission 125 du mouvement aux éléments de massage, connecté au moteur 3 par le biais d'un système de connexion qui permet de s'affranchir de l'angle d'inclinaison de la tête de massage par rapport au corps2. Cette pièce 124 comprend des crans 126, 127 et des moyens de rétractation 128, 129 des crans 126, 127 permettant de les escamoter pour leur positionnement dans les encoches 122, 123 et aussi leur retrait de ces encoches 122, 123.

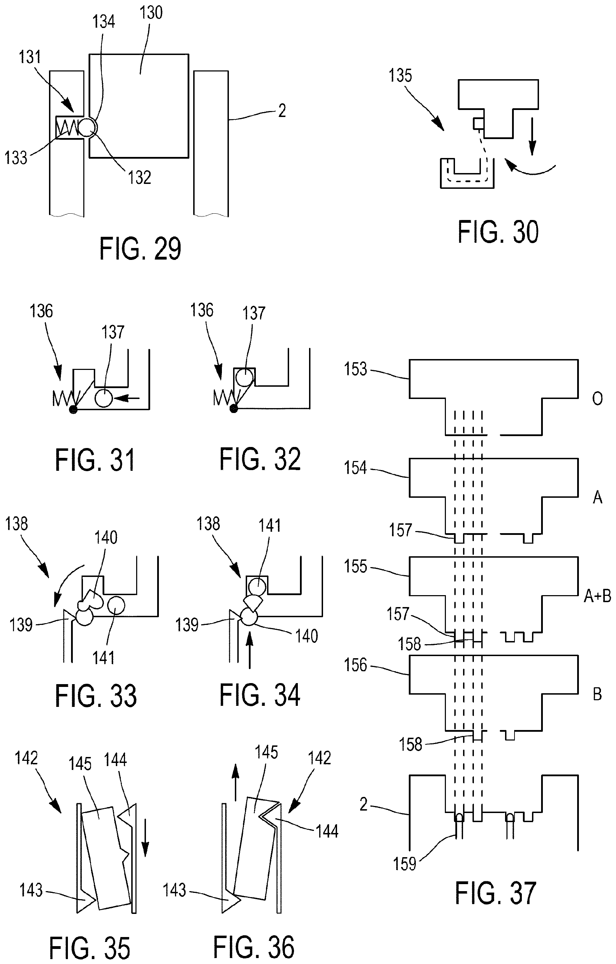

Différents systèmes d'encliquetage ou moyens de rétractation sont illustrés sur les figures 29 à 36 est permettent de maintenir assembler une tête de massage 130 sur le corps 2. Sur la figure 29 un système de butée 131 à bille 132 est illustré, la bille 132 étant montée sur ressort 133 et s'engageant dans une gorge 134 sur la tête de massage 130. Sur la figure 30, un système à baïonnette 135 est illustré. Sur les figures 31 et 32 est illustré un système à clapet et ressort 136 permettant le blocage d'une pièce 137 assujettie à la tête de massage. Sur les figures 33 et 34, un système de blocage 138 avec cran 139 et loquet de déblocage 140 est illustré dans une position de blocage et de déblocage d'une pièce 141. Sur les figures 35 et 36, un système de blocage 142 permet à deux crans 143, 144 de bloquer ou de débloquer une pièce 145. Ces différentes variantes de réalisation ne sont pas limitatives.

Tel qu'illustré en figures 14 à 20, l'appareil de massage 1 comprend des moyens de reconnaissance 146 de la tête de massage 147 assemblée sur le corps 2.

Sur les figures 14 et 15, la tête de massage 146 comprend une protubérance 148 de forme circulaire qui est prévue pour être engagée dans une rainure circulaire 149 sur le corps 2. Le corps 2 comprend une seconde rainure circulaire 150. Dans ces rainures circulaires 149, 150 sont agencés des contacteurs mécaniques 151, 152. Lorsque la tête de massage 146 est assemblée sur le corps 2, la protubérance 148 actionne le contacteur mécanique 151. On constate sur la figure 37 quatre têtes de massage 153, 154, 155, 156 qui peuvent être assemblées sur le corps 2. La première tête de massage 153 ne comprend aucune protubérance. La seconde tête de massage 154 comprend une protubérance 157. La troisième tête de massage 155 comprend deux protubérances 157, 158. Et la quatrième tête de massage 156 comprend une protubérance 158. Lors de l'assemblage de ces têtes de massage 153, 154, 155, 156 sur le corps 2, elles actionnent l'un et/ou l'autre des deux contacteurs mécaniques 159, 160, ce qui permet leur reconnaissance en fonction du codage binaire programmé sur un boîtier de commande 161 illustré en figure 14. Ce boîtier de commande 161 est configuré pour contrôler les différents actionneurs présents dans l'appareil de massage 1 en fonction du type de tête de massage assemblée. Ainsi, par exemple, le boîtier de commande 161 permet de contrôler l'actionnement des moteurs, des pompes de distribution de produit cosmétique, du dispositif de traitement ionophorèse transcutanée, du système vibratoire, du système luminescent ...

Sur la figure 16, on constate que les protubérances 162, 163 sont disposées sur le corps 2 et mises en œuvre par les contacteurs mécaniques 164, 165. Tandis que la tête de massage 147 comprend une rainure circulaire 166.

Des variantes sont envisageables dans le cadre de l'invention. On peut notamment remplacer les contacteurs mécaniques par des contacteurs ou des capteurs magnétiques.

Sur les figures 14 et 17 à 20 sont représentés des capteurs optiques 167. Sur la figure 17, la tête de massage 147 comprend une surface transparente 168 qui laisser passer la lumière émise par une diode luminescente 169 et détectée par le capteur optique 170. On détecte ainsi la lumière par transparence. Sur la variante de la figure 18, la surface 171 est opaque, le capteur 170 détecte donc une opacité lors de l'assemblage de la tête de massage 147. Sur la figure 19, un séparateur 172 est agencé entre la tête de massage 147 et le corps 2. Sur la figure 20, un système de miroir est agencé entre la tête de massage 147 et le corps 2.

D'autres variantes sont envisageables sans sortir du cadre de l'invention, notamment quant à la reconnaissance des têtes de massage sur le corps 2 et au boîtier de commande 161. Les capteurs pourront par exemple permettre de détecter l'assemblage convenable de la tête de massage 147 sur le corps 2.

## Revendications

1. Appareil de massage (1) de la peau comprenant :
- un corps (2) qui comporte des moyens de motorisation formés par un moteur (3) alimenté électriquement,
- au moins un type de tête de massage (4, 5, 6, 7, 8) qui comprend des éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps et des moyens de commande (161) dudit appareil de massage en fonction de la reconnaissance du type de tête de massage, **caractérisé en ce que** l'appareil de massage (1) comprend également un moto réducteur agencé en sortie du moteur (3),
dans lequel la tête de massage (7) comporte une surface d'application (25) et les éléments de massage sont constitués de trois embouts de massage inclinés vers l'extérieur par rapport à la surface d'application, le mécanisme de transmission étant configuré pour actionner en rotation les trois embouts de massage selon trois axes fixes respectifs, perpendiculaires à la surface d'application.

2. Appareil de massage (1) de la peau comprenant :
- un corps (2) qui comporte des moyens de motorisation formés par un moteur (3) alimenté électriquement,
- au moins un type de tête de massage (4, 5, 6, 7, 8) qui comprend des éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps et des moyens de commande (161) dudit appareil de massage en fonction de la reconnaissance du type de tête de massage, **caractérisé en ce que** l'appareil de massage (1) comprend également un moto réducteur agencé en sortie du moteur (3),
dans lequel la tête de massage (5) comporte une surface d'application (21) et les éléments de massage sont constitués d'au moins deux embouts de massage qui s'étendent perpendiculairement vers l'extérieur de la surface d'application, les embouts de massage étant définis sur la surface d'application selon des cercles virtuels concentriques de centre C et, dans lequel mécanisme de transmission est configuré pour rapprocher les embouts de massage et/ou, inversement, pour écarter les embouts de massage, en translatant lesdits embouts de massage dans un sens et/ou dans l'autre selon des trajectoires qui se rejoignent au centre C.

3. Appareil de massage (1) de la peau comprenant :
- un corps (2) qui comporte des moyens de motorisation formés par un moteur (3) alimenté électriquement,
- au moins un type de tête de massage (4, 5, 6, 7, 8) qui comprend des éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps et des moyens de commande (161) dudit appareil de massage en fonction de la reconnaissance du type de tête de massage, **caractérisé en ce que** l'appareil de massage (1) comprend également un moto réducteur agencé en sortie du moteur (3),
dans lequel la tête de massage (6) comporte une surface d'application (23) et les éléments de massage sont constitués de deux embouts de massage qui s'étendent perpendiculairement vers l'extérieur de la surface d'application, le mécanisme de transmission étant configuré pour actionner en rotation selon un axe perpendiculaire à la surface d'application, avec un mouvement oscillatoire, les deux embouts.

4. Appareil de massage (1) de la peau comprenant :
- un corps (2) qui comporte des moyens de motorisation formés par un moteur (3) alimenté électriquement,
- au moins un type de tête de massage (4, 5, 6, 7, 8) qui comprend des éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps et des moyens de commande (161) dudit appareil de massage en fonction de la reconnaissance du type de tête de massage, **caractérisé en ce que** l'appareil de massage (1) comprend également un moto réducteur agencé en sortie du moteur (3),
dans lequel la tête de massage (8) comprend une surface d'application et les éléments de massage sont constitués d'au moins deux doigts de massage qui s'étendent vers l'extérieur de la surface d'application, les doigts de massage et le mécanisme de transmission étant configurés pour former une pince.

5. Appareil de massage (1) selon l'une des revendications 1 à 4, dans lequel la tête de massage (5, 6, 7, 8) comprend une peau souple (29) qui recouvre les éléments de massage (22a, 22b, 22c, 24a, 24b, 26a, 26b, 26c, 28a, 28b).

6. Appareil de massage (1) selon l'une des revendications 1 à 4, dans lequel la tête de massage comprend une couronne (56) s'étendant perpendiculairement à la surface d'application et, le mécanisme de transmission est configuré pour entraîner la couronne avec un mouvement oscillatoire autour d'un axe perpendiculaire à la surface d'application.

7. Appareil de massage (1) de la peau comprenant :
- un corps (2) qui comporte des moyens de motorisation formés par un moteur (3) alimenté électriquement,
- au moins un type de tête de massage (4, 5, 6, 7, 8) qui comprend des éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps et des moyens de commande (161) dudit appareil de massage en fonction de la reconnaissance du type de tête de massage, **caractérisé en ce que** l'appareil de massage (1) comprend également un moto réducteur agencé en sortie du moteur (3),
dans lequel la tête de massage (4) comprend une surface d'application (31) et les éléments de massage sont constitués de deux rouleaux de massage agencés selon deux axes longitudinaux parallèles, avec un écartement entre eux, et dépassant partiellement à l'extérieur de la surface d'application et, dans lequel mécanisme de transmission est configuré pour entraîner en rotation de manière synchronisée et inversée, les deux rouleaux de sorte que, dans une vue en plan perpendiculaire aux deux axes, la partie du rouleau de massage située à gauche qui dépasse de la surface d'application, tourne dans le sens trigonométrique et, la partie du rouleau de massage située à droite qui dépasse de la surface d'application, tourne dans le sens horaire.

8. Appareil de massage (1) selon l'une des revendications 1 à 7, dans lequel les moyens de reconnaissance (13) sont des capteurs mécaniques (151, 152, 164, 165) agencés sur l'au moins un type de tête de massage (147) et le corps (2) et, configurés pour transmettre des informations aux moyens de commande (161) en fonction du type de tête de massage assemblé sur le corps.

9. Appareil de massage (1) selon l'une des revendications 1 à 7, dans lequel les moyens de reconnaissance (146) sont des capteurs magnétiques agencés sur l'au moins un type de tête de massage (147) et le corps (2) et, configurés pour transmettre des informations aux moyens de commande (161) en fonction du type de tête de massage assemblé sur le corps.

10. Appareil de massage (1) selon l'une des revendications 1 à 7, dans lequel les moyens de reconnaissance (146) sont des capteurs optiques (169, 170) agencés sur l'au moins un type de tête de massage (147) et le corps (2) et, configurés pour transmettre des informations aux moyens de commande (161) en fonction du type de tête de massage assemblé sur le corps.

11. Appareil de massage (1) selon l'une des revendications 1 à 10, dans lequel les moyens de commande (161) sont configurés pour agir sur les moyens de motorisation (3) et modifier leur actionnement en fonction du type de tête de massage (4, 5, 6, 7, 8) assemblé sur le corps (2).

12. Appareil de massage (1) selon l'une des revendications 1 à 11, lequel comprend un système d'émission d'ondes (106) agencé sur le corps et des moyens de transfert (108) des ondes, du système d'émission vers la tête de massage (4, 5, 6, 7, 8).

13. Appareil de massage (1) selon la revendication 12, dans lequel les moyens de commande (161) sont configurés pour agir sur le système d'émission d'ondes (106) et modifier les ondes en fonction du type de tête de massage (4, 5, 6, 7, 8) assemblé sur le corps (2).

14. Appareil de massage (1) selon l'une des revendications 1 à 13, lequel comprend un système de distribution de produit cosmétique (77) agencé sur le corps et des moyens de transfert (90) du produit, du corps (2) vers la tête de massage (4, 5, 6, 7, 8).

15. Appareil de massage (1) selon la revendication 14, lequel comprend des moyens d'actionnement (91) du système de distribution de produit cosmétique configurés pour permettre le fonctionnement dudit système de distribution en mode manuel ou en mode automatique.

16. Appareil de massage (1) selon l'une des revendications 14 ou 15, dans lequel les moyens de commande (161) sont configurés pour agir sur le système de distribution de produit cosmétique et modifier la distribution du produit cosmétique en fonction du type de tête de massage (4, 5, 6, 7, 8) assemblé sur le corps (2).

17. Appareil de massage (1) selon l'une des revendications 1 à 16, dans lequel les moyens d'assemblage (9) comprennent un système de détection de l'assemblage convenable de la tête de massage (4, 5, 6, 7, 8) sur le corps (2).

18. Appareil de massage (1) selon l'une des revendications 1 à 17, lequel comprend des moyens de connexion entre les moyens de motorisation (3) et le mécanisme de transmission configurés pour s'affranchir de l'angle d'orientation du mécanisme de transmission par rapport au moyen de motorisation, lors de l'assemblage de la tête de massage (4, 5, 6, 7, 8) sur le corps (2).

19. Appareil de massage (1) selon l'une des revendications 1 à 18, lequel comprend un dispositif de traitement ionophorèse transcutanée qui est configuré pour transmettre sur la peau lors de l'application dudit appareil de massage, un courant permettant d'augmenter et/ou d'accélérer la pénétration d'un produit cosmétique.

## Patentansprüche

1. Massagegerät (1) für die Haut, umfassend:
- einen Körper (2), der Motorisierungsmittel aufweist, die von einem elektrisch versorgten Motor (3) gebildet sind,
- mindestens einen Typ von Massagekopf (4, 5, 6, 7, 8), der Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes, und Steuermittel (161) des Massagegeräts in Abhängigkeit von der Erkennung des Typs des Massagekopfs, **dadurch gekennzeichnet, dass** das Massagegerät (1) ebenfalls einen Getriebemotor umfasst, der am Ausgang des Motors (3) eingerichtet ist,
wobei der Massagekopf (7) eine Applikationsfläche (25) aufweist und die Massageelemente aus drei im Verhältnis zu der Applikationsfläche nach außen geneigten Massagespitzen bestehen, wobei der Übertragungsmechanismus so konfiguriert ist, dass er die drei Massagespitzen in drei jeweiligen festen Achsen dreht, die senkrecht zu der Applikationsfläche sind.

2. Massagegerät (1) für die Haut, umfassend:
- einen Körper (2), der Motorisierungsmittel aufweist, die von einem elektrisch versorgten Motor (3) gebildet sind,
- mindestens einen Typ von Massagekopf (4, 5, 6, 7, 8), der Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes, und Steuermittel (161) des Massagegeräts in Abhängigkeit von der Erkennung des Typs des Massagekopfs, **dadurch gekennzeichnet, dass** das Massagegerät (1) ebenfalls einen Getriebemotor umfasst, der am Ausgang des Motors (3) eingerichtet ist,
wobei der Massagekopf (5) eine Applikationsfläche (21) aufweist und die Massageelemente aus mindestens zwei Massagespitzen bestehen, die sich senkrecht nach außerhalb der Applikationsfläche erstrecken, wobei die Massagespitzen auf der Applikationsfläche in konzentrischen virtuellen Kreisen mit Zentrum C definiert sind, und wobei der Übertragungsmechanismus dazu ausgelegt ist, die Massagespitzen anzunähern und/oder umgekehrt, die Massagespitzen zu beabstanden, durch Verschieben der Massagespitzen in einer Richtung und/oder in der anderen auf Wegen, die sich im Zentrum C treffen.

3. Massagegerät (1) für die Haut, umfassend:
- einen Körper (2), der Motorisierungsmittel aufweist, die von einem elektrisch versorgten Motor (3) gebildet sind,
- mindestens einen Typ von Massagekopf (4, 5, 6, 7, 8), der Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes, und Steuermittel (161) des Massagegeräts in Abhängigkeit von der Erkennung des Typs des Massagekopfs, **dadurch gekennzeichnet, dass** das Massagegerät (1) ebenfalls einen Getriebemotor umfasst, der am Ausgang des Motors (3) eingerichtet ist,
wobei der Massagekopf (6) eine Applikationsfläche (23) aufweist und die Massageelemente aus zwei Massagespitzen bestehen, die sich senkrecht nach außerhalb der Applikationsfläche erstrecken, wobei der Übertragungsmechanismus dazu ausgelegt ist, die zwei Spitzen mit einer schwingenden Bewegung in einer Achse senkrecht zur Applikationsfläche drehend anzutreiben.

4. Massagegerät (1) für die Haut, umfassend:
- einen Körper (2), der Motorisierungsmittel aufweist, die von einem elektrisch versorgten Motor (3) gebildet sind,
- mindestens einen Typ von Massagekopf (4, 5, 6, 7, 8), der Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes, und Steuermittel (161) des Massagegeräts in Abhängigkeit von der Erkennung des Typs des Massagekopfs, **dadurch gekennzeichnet, dass** das Massagegerät (1) ebenfalls einen Getriebemotor umfasst, der am Ausgang des Motors (3) eingerichtet ist,
wobei der Massagekopf (8) eine Applikationsfläche aufweist und die Massageelemente aus mindestens zwei Massagefingern bestehen, die sich nach außerhalb der Applikationsfläche erstrecken, wobei die Massagefinger und der Übertragungsmechanismus dazu ausgelegt sind, eine Zange zu bilden.

5. Massagegerät (1) nach einem der Ansprüche 1 bis 4, wobei der Massagekopf (5, 6, 7, 8) eine elastische Haut (29) umfasst, die die Massageelemente (22a, 22b, 22c, 24a, 24b, 26a, 26b, 26c, 28a, 28b) bedeckt.

6. Massagegerät (1) nach einem der Ansprüche 1 bis 4, wobei der Massagekopf einen Ring (56) umfasst, der sich senkrecht zu Applikationsfläche erstreckt und der Übertragungsmechanismus derart ausgelegt ist, dass er den Ring mit einer schwingenden Bewegung um eine Achse senkrecht zur Applikationsfläche antreibt.

7. Massagegerät (1) für die Haut, umfassend:
- einen Körper (2), der Motorisierungsmittel aufweist, die von einem elektrisch versorgten Motor (3) gebildet sind,
- mindestens einen Typ von Massagekopf (4, 5, 6, 7, 8), der Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes, und Steuermittel (161) des Massagegeräts in Abhängigkeit von der Erkennung des Typs des Massagekopfs, **dadurch gekennzeichnet, dass** das Massagegerät (1) ebenfalls einen Getriebemotor umfasst, der am Ausgang des Motors (3) eingerichtet ist,
wobei der Massagekopf (4) eine Applikationsfläche (31) umfasst und die Massageelemente aus zwei Massagerollen bestehen, die entlang zweier paralleler Längsachsen mit einem Abstand voneinander angeordnet sind und teilweise aus der Applikationsfläche herausragen, und wobei ein Übertragungsmechanismus so konfiguriert ist, dass er die beiden Rollen synchron und in umgekehrter Richtung dreht, so dass in einer Draufsicht senkrecht zu den beiden Achsen der Teil der Massagerolle, der sich links befindet und von der Applikationsfläche herausragt, sich in dem trigonometrischen Sinn dreht und der Teil der Massagerolle, der sich rechts befindet und von der Applikationsfläche herausragt, sich im Uhrzeigersinn dreht.

8. Massagegerät (1) nach einem der Ansprüche 1 bis 7, wobei die Erkennungsmittel (13) mechanische Sensoren (151, 152, 164, 165) sind, die auf dem mindestens einen Typ von Massagekopf (147) und dem Körper (2) eingerichtet sind und dazu ausgelegt, Informationen an die Steuermittel (161) in Abhängigkeit von dem auf dem Körper angebrachten Massagekopftyp zu übertragen.

9. Massagegerät (1) nach einem der Ansprüche 1 bis 7, wobei die Erkennungsmittel (146) magnetische Sensoren sind, die auf dem mindestens einen Typ von Massagekopf (147) und dem Körper (2) eingerichtet sind und dazu ausgelegt, Informationen an die Steuermittel (161) in Abhängigkeit von dem auf dem Körper angebrachten Massagekopftyp zu übertragen.

10. Massagegerät (1) nach einem der Ansprüche 1 bis 7, wobei die Erkennungsmittel (146) optische Sensoren (169, 170) sind, die auf dem mindestens einen Typ von Massagekopf (147) und dem Körper (2) eingerichtet sind und dazu ausgelegt, Informationen an die Steuermittel (161) in Abhängigkeit von dem auf dem Körper angebrachten Massagekopftyp zu übertragen.

11. Massagegerät (1) nach einem der Ansprüche 1 bis 10, wobei die Steuermittel (161) so konfiguriert sind, dass sie auf die Motormittel (3) wirken und deren Betätigung von Abhängigkeit von dem auf dem Körper (2) angebrachten Typ des Massagekopfs (4, 5, 6, 7, 8) ändern.

12. Massagegerät (1) nach einem der Ansprüche 1 bis 11, das ein System zum Aussenden von Wellen (106), das auf dem Körper (2) angeordnet ist, und Mittel zur Übertragung der Wellen (108) von dem System zum Aussenden zu dem Massagekopf (4, 5, 6, 7, 8) umfasst.

13. Massagegerät (1) nach Anspruch 12, wobei die Steuermittel (161) so konfiguriert sind, dass sie auf das System zum Aussenden von Wellen (106) einwirken und die Wellen in Abhängigkeit von dem Typ des an dem Körper (2) montierten Massagekopfes (4, 5, 6, 7, 8) ändern.

14. Massagegerät (1) nach einem der Ansprüche 1 bis 13, umfassend ein an dem Körper angeordnetes Abgabesystem (77) für ein kosmetisches Produkt und Mittel zur Übertragung (90) des Produkts von dem Körper (2) zu dem Massagekopf (4, 5, 6, 7, 8).

15. Massagegerät (1) nach Anspruch 14, das Betätigungsmittel (91) des Abgabesystems für ein kosmetisches Produkt umfasst, die dazu ausgelegt sind, den Betrieb des Abgabesystems im manuellen Modus oder im automatischen Modus zu gestatten.

16. Massagegerät (1) nach einem der Ansprüche 14 oder 15, wobei die Steuermittel (161) so konfiguriert sind, dass sie auf das System zur Abgabe eines kosmetischen Produkts einwirken und die Abgabe des kosmetischen Produkts in Abhängigkeit von dem Typ des auf dem Körper (2) montierten Massagekopfes (4, 5, 6, 7, 8) ändern.

17. Massagegerät (1) nach einem der Ansprüche 1 bis 16, wobei die Montagemittel (9) ein System zur Detektion der korrekten Montage des Massagekopfes (4, 5, 6, 7, 8) auf dem Körper (2) umfassen.

18. Massagegerät (1) nach einem der Ansprüche 1 bis 17, der Verbindungsmittel zwischen den Motormitteln (3) und dem Übertragungsmechanismus umfasst, die dazu ausgelegt sind, sich bei der Montage des Massagekopfes (4, 5, 6, 7, 8) auf dem Körper (2) von dem Ausrichtungswinkel des Übertragungsmechanismus im Verhältnis zum Motormittel freizumachen.

19. Massagegerät (1) nach einem der Ansprüche 1 bis 18, das eine Vorrichtung zur transkutanen Iontophorese-Behandlung umfasst, die so konfiguriert ist, dass sie bei Anwendung des Massagegeräts einen Strom, der die Erhöhung und/oder Beschleunigung der Penetration eines kosmetischen Produkts ermöglicht, auf die Haut überträgt.

## Claims

1. A massaging appliance (1) for skin, comprising:
- a body (2) that includes driving means formed by an electrically powered motor (3),
- at least one type of massaging head (4, 5, 6, 7, 8) that includes massaging elements,
- a transmission mechanism that activates the massaging elements via the propulsion of the driving means,
- means of attachment (9) that are configured to attach, in a removable manner, the at least one type of massaging head to the body;
- means of distinguishing (146) the type of massaging head attached to the body, and control means (161) of said massaging appliance depending on the type of massaging head distinguished, **characterized in that** the massaging appliance (1) also comprises a gear unit arranged at the output of the motor (3) ;
wherein the massaging head (7) comprises an application surface (25) and the massaging elements consist of three massaging endpieces inclined outwardly from the application surface, the transmission mechanism being configured for rotatably actuating the three massaging endpieces along three respective fixed axes perpendicular to the application surface.

2. A massaging appliance (1) for skin, comprising:
- a body (2) that includes driving means formed by an electrically powered motor (3);
- at least one type of massaging head (4,5 6,7,8) that includes massaging elements;
- a transmission mechanism that activates the massaging elements via the propulsion of the driving means,
- means of attachment (9) that are configured to attach, in a removable manner, the at least one type of massaging head to the body;
- means of distinguishing (146) the type of massaging head attached to the body, and control means (161) of said massaging appliance depending on the type of massaging head distinguished, **characterized in that** the massaging appliance (1) also comprises a gear unit arranged at the output of the motor (3),
wherein the massaging head (5) comprises an application surface (21) and the massaging elements consist of at least two massaging endpieces extending perpendicularly outwardly from the application surface, the massaging endpieces being defined on the application surface in virtual concentric circles having a centre C, and wherein the transmission mechanism is configured to draw together the massaging endpieces and/or conversely to draw apart the massaging endpieces by translating said massaging endpieces in one direction and/or in the other along trajectories which meet at the centre C.

3. A massaging appliance (1) for skin, comprising:
- a body (2) that includes driving means formed by an electrically powered motor (3),
- at least one type of massaging head (4,5 6,7,8) that includes massaging elements,
- a transmission mechanism that activates the massaging elements via the propulsion of the driving means,
- means of attachment (9) that are configured to attach, in a removable manner, the at least one type of massaging head to the body,
- means of distinguishing (146) the type of massaging head attached to the body, and control means (161) of said massaging appliance depending on the type of massaging head distinguished, **characterized in that** the massaging appliance (1) also comprises a gear unit arranged at the output of the motor (3),
wherein the massaging head (6) comprises an application surface (23) and the massaging elements are composed of two massaging endpieces extending perpendicularly outwardly from the application surface, the transmission mechanism being configured rotatably to activate the two endpieces along an axis perpendicular to the application surface, with an oscillatory movement.

4. A massaging appliance (1) for skin, comprising:
- a body (2) that includes driving means formed by an electrically powered motor (3),
- at least one type of massaging head (4, 5 6, 7, 8) that includes massaging elements,
- a transmission mechanism that activates the massaging elements via the propulsion of the driving means,
- means of attachment (9) that are configured to attach, in a removable manner, the at least one type of massaging head to the body;
- means of distinguishing (146) the type of massaging head attached to the body, and control means (161) of said massaging appliance depending on the type of massaging head distinguished, **characterized in that** the massaging appliance (1) also comprises a gear unit arranged at the output of the motor (3),
wherein the massaging head (8) comprises an application surface and the massaging elements consist of at least two massaging fingers extending outwardly from the application surface, the massaging fingers and the transmission mechanism being configured to form a pincer.

5. The massaging appliance (1) according to one of claims 1 to 4, wherein the massaging head (5, 6, 7, 8) comprises a soft skin (29) covering the massaging elements (22a, 22b, 22c, 24a, 24b, 26a, 26b, 26c, 28a, 28b).

6. The massaging appliance (1) according to one of claims 1 to 4, wherein the massaging head comprises a crown (56) extending perpendicular to the application surface, and the transmission mechanism is configured to drive the crown in an oscillatory movement about an axis perpendicular to the application surface.

7. A massaging appliance (1) for skin, comprising:
- a body (2) that includes driving means formed by an electrically powered motor (3);
- at least one type of massaging head (4, 5, 6, 7, 8) that includes massaging elements;
- a transmission mechanism that activates the massaging elements via the propulsion of the driving means,
- means of attachment (9) that are configured to attach, in a removable manner, the at least one type of massaging head to the body;
- means of distinguishing (146) the type of massaging head attached to the body, and control means (161) of said massaging appliance depending on the type of massaging head distinguished, **characterized in that** the massaging appliance (1) also comprises a gear unit arranged at the output of the motor (3),
wherein the massaging head (4) comprises an application surface (31) and the massaging elements consist of two massaging rollers positioned along two parallel longitudinal axes, with a gap between them, and partially extending beyond the exterior of the application surface, and in which the transmission mechanism is configured to spin the two rollers in an inverse synchronised manner such that, when viewed along a plane perpendicular to the two axes, the portion of massaging roller located on the left which extends beyond the application surface spins in the trigonometric direction, and the portion of the massaging roller located on the right that extends beyond the application surface spins clockwise.

8. The massaging appliance (1) according to one of claims 1 to 7, wherein the distinguishing means (13) are mechanical sensors (151, 152, 164, 165) arranged on the at least one type of massaging head (147) and the body (2), and are configured to transmit information to the control means (161) depending on the type of massaging head attached to the body.

9. The massaging appliance (1) according to one of claims 1 to 7, wherein the distinguishing means (146) are magnetic sensors arranged on the at least one type of massaging head (147) and the body (2), and are configured to transmit information to the control means (161) depending on the type of massaging head attached to the body.

10. The massaging appliance (1) according to one of claims 1 to 7, wherein the distinguishing means (146) are optical sensors (169, 170) arranged on the at least one type of massaging head (147) and the body (2), and are configured to transmit information to the control means (161) depending on the type of massaging head attached to the body.

11. The massaging appliance (1) according to one of claims 1 to 10, wherein the control means (161) are configured to act upon the driving means (3) and to modify activation thereof depending on the type of massaging head (4, 5, 6, 7, 8) attached to the body (2).

12. The massaging appliance (1) according to one of claims 1 to 11 with a means for emitting waves (106) arranged on the body, and transfer means (108) of the waves from the emitting means to the massaging head (4, 5, 6, 7, 8).

13. The massaging appliance (1) according to claim 12, wherein the control means (161) are configured to act upon the wave-emitting means (106) and to modify the waves depending on the type of massaging head (4, 5, 6, 7, 8) attached to the body (2) .

14. The massaging appliance (1) according to one of claims 1 to 13, which comprises a cosmetic product dispensing system (77) located on the body, and a means of transfer (90) of the product from the body (2) to the massaging head (4, 5, 6, 7, 8) .

15. The massaging appliance (1) according to claim 14 comprising activation means (91) of the cosmetic product dispensing system configured to allow functioning of said dispensing system in manual mode or automatic mode.

16. The massaging appliance (1) according to one of claims 14 or 15, wherein the control means (161) are configured to act upon the cosmetic product dispensing system and to modify dispensing of the cosmetic product depending on the type of massaging head (4, 5, 6, 7, 8) attached to the body (2).

17. The massaging appliance (1) according to one of claims 1 to 16, wherein the attachment means (9) comprise a system for detecting suitable assembling of the massaging head (4, 5, 6, 7, 8) on the body (2).

18. The massaging appliance (1) according to one of claims 1 to 17, which comprises connection means between the driving means (3) and the transmission mechanism, configured to obviate the need for an angle of orientation of the transmission mechanism in relation to the driving means when assembling the massaging head (4, 5, 6, 7, 8) on the body (2).

19. The massaging appliance (1) according to one of claims 1 to 18 which comprises a transcutaneous ionophoresis treatment device which is configured to transmit to the skin, while said massaging appliance is applied, a current that improves and/or accelerates the penetration of a cosmetic product.
